# FASCICULE DE BREVET EUROPEEN

(11) **EP 1 945 140 B1**
(45) Date de publication et mention de la délivrance du brevet: **13.05.2015**
(21) Numéro de dépôt: 06831099.4
(22) Date de dépôt: 09.11.2006
(51) Int. Cl.: A61F 2/16

(54) **INJECTEUR ET CARTOUCHE DE PLIAGE D'IMPLANT OPHTALMIQUE**
INJEKTOR UND ZUSAMMENKLAPPBARE KARTUSCHE FÜR EIN AUGENIMPLANTAT
OPHTHALMIC IMPLANT INJECTOR AND FOLDING CARTRIDGE

(30) Priorité: 09.11.2005 FR 0511421
(43) Date de publication de la demande: 23.07.2008
(73) Titulaire: SEDAT, 69540 Irigny (Rhône) (FR)
(72) Inventeur: PESSIN, Olivier, F-69290 Grezieu La Varenne (FR)
(74) Mandataire: Blot, Philippe Robert Emile
(86) Numéro de dépôt international: PCT/FR2006/002499
(87) Numéro de publication internationale: WO 2007/054645

(56) Documents cités:
- WO-A-02/074208
- WO-A-03/044946
- WO-A-2005/082284
- WO-A-2005/082285
- US-A- 6 142 999

## Description

La présente invention concerne une cartouche de pliage d'un implant ophtalmique.

Il est connu pour le traitement de la cataracte qu'un chirurgien injecte dans l'oeil du patient un implant ophtalmique ayant par exemple la forme d'une lentille équipée latéralement de deux bras arqués diamétralement opposés.

L'implant est injecté dans l'oeil du patient par l'intermédiaire d'une canule d'injection, en étant poussé par un piston au travers de cette canule. Pour le passage au travers de la canule, l'implant est enroulé sur lui-même en spirale autour d'une direction diamétrale reliant les deux bras de l'implant.

L'enroulement de l'implant sous forme de spirale est désigné par pliage et est effectué dans une cartouche de pliage. Cette cartouche comporte la canule d'injection et est adaptée pour être rapportée après chargement sur un mécanisme d'injection comportant un corps de support et un poussoir coulissant.

Les cartouches de pliage connues (par example WO-2005/0822 84 A1) comportent deux branches articulées l'une par rapport à l'autre pour former une pince. Chaque branche présente au voisinage de l'articulation un canal semi-cylindrique s'étendant parallèlement à l'axe d'articulation. Les canaux des deux branches s'ouvrent en regard l'un de l'autre de sorte que lorsque les deux branches sont accolées, les canaux forment un conduit de confinement de l'implant. La section semi-circulaire des canaux permet lors du rapprochement des deux branches, de replier la lentille constituant l'implant sur elle-même de sorte que les bords extrêmes se trouvent rapprochés en regard l'un de l'autre.

La poursuite de l'enroulement de l'implant sur lui-même pour former une spirale s'effectue en poussant l'implant au travers de la canule, laquelle canule présente à cet effet une surface intérieure tronconique dont le diamètre varie du conduit de confinement de l'implant jusqu'à l'extrémité de sortie.

Ainsi, l'enroulement en spirale de l'implant est initié lors du rapprochement des deux branches alors que l'implant est maintenu entre les deux canaux.

Les deux canaux assurent un rapprochement des bords opposés de l'implant de sorte que celui-ci forme sensiblement un cylindre. Pour former une spirale, il convient que les deux bords opposés se chevauchent, l'un passant à l'intérieur et l'autre passant à l'extérieur. Ce chevauchement s'effectue aléatoirement dans un sens ou dans l'autre, notamment en fonction du positionnement initial de l'implant par le chirurgien entre les deux canaux.

Par ailleurs, dans certaines circonstances, les deux bords se trouvent rigoureusement en regard l'un de l'autre lorsque les deux branches sont accolées de sorte que lorsque l'implant pénètre dans la canule à profil intérieur tronconique, les deux bords sont comprimés l'un contre l'autre, ce qui peut provoquer un endommagement de l'implant.

L'invention a pour but de proposer une cartouche de pliage évitant l'endommagement de l'implant lors de sa phase de pliage.

A cet effet, l'invention a pour objet une cartouche de pliage du type précité, caractérisée en ce que en position accolée des deux branches, au moins deux des rives en regard des deux canaux sont décalés transversalement au conduit dans le plan de jonction des deux branches de sorte qu'une branche délimite un épaulement d'appui de l'implant faisant saillie en regard du canal ménagé dans l'autre branche.

Selon des modes particuliers de réalisation, la cartouche comporte l'une ou plusieurs des caractéristiques suivantes :
- l'épaulement a dans le plan de jonction une largeur moyenne mesurée transversalement à la direction des canaux comprise entre 0,1 et 0,8 mm;
- les deux rives décalées sont plus éloignées de l'axe d'articulation des deux branches que les deux autres rives, lesquelles autres rives sont confondues lorsque les deux branches sont accolées ;
- la cartouche comporte une canule d'injection à surface interne convergente dans le prolongement du conduit de confinement de l'implant lorsque les deux branches sont accolées ;
- les deux canaux sont de section sensiblement semi-circulaire ou semi-elliptique ;
- l'épaulement est sensiblement plan et l'axe du conduit formé des deux canaux s'étend sensiblement dans le plan défini par l'épaulement;
- chaque branche présente au-delà du canal, du côté opposé à l'axe d'articulation, une aile de manoeuvre et l'épaulement prolonge une aile de manoeuvre de l'une des branches ; et
- les deux branches comportent, au voisinage des rives des canaux, en dehors des canaux, des profils d'emboîtement complémentaires en saillie et en creux dimensionnés de sorte que les profils sont emboîtés lorsque les deux branches sont accolées.

L'invention a également pour objet un injecteur d'implant comportant une cartouche telle que définie ci-dessus et un mécanisme d'injection comprenant un corps de liaison à la cartouche et un poussoir propre à pousser l'implant contenu dans la cartouche.

L'invention sera mieux comprise à la lecture de la description qui va suivre, donnée uniquement à titre d'exemple et faite en se référant aux dessins sur lesquels :
- la Figure 1 est une vue en perspective d'un injecteur d'implant ophtalmique équipé d'une cartouche prêt pour une injection ;
- la Figure 2 est une vue en perspective de l'injecteur de la Figure 1, la cartouche étant partiellement recouverte de son étui de protection ;
- la Figure 3 est une vue en perspective éclatée de la cartouche de pliage ouverte, de l'étui de protection et du piston ;
- la Figure 4 est une vue en perspective de la cartouche de pliage reçue partiellement dans l'étui avec les deux branches ouvertes et un implant avant déformation maintenu entre les deux branches;
- la Figure 5 est une vue en coupe transversale de la cartouche prise suivant le plan V-V de la Figure 4 ;
- la Figure 6 est une vue en coupe de la cartouche prise suivant la ligne VI-VI de la Figure 7 ;
- la Figure 6A est une vue agrandie d'une partie de la Figure 6 ;
- la Figure 7 est une vue en élévation de la cartouche de pliage re-fermée ;
- la Figure 8 est une vue en section de la cartouche prise suivant la ligne VIII-VIII de la Figure 6 lors du déplacement de l'implant ; et
- la Figure 9 est une vue en section transversale de la cartouche de pliage, lors du rapprochement des deux branches montrant un mauvais positionnement de l'implant.

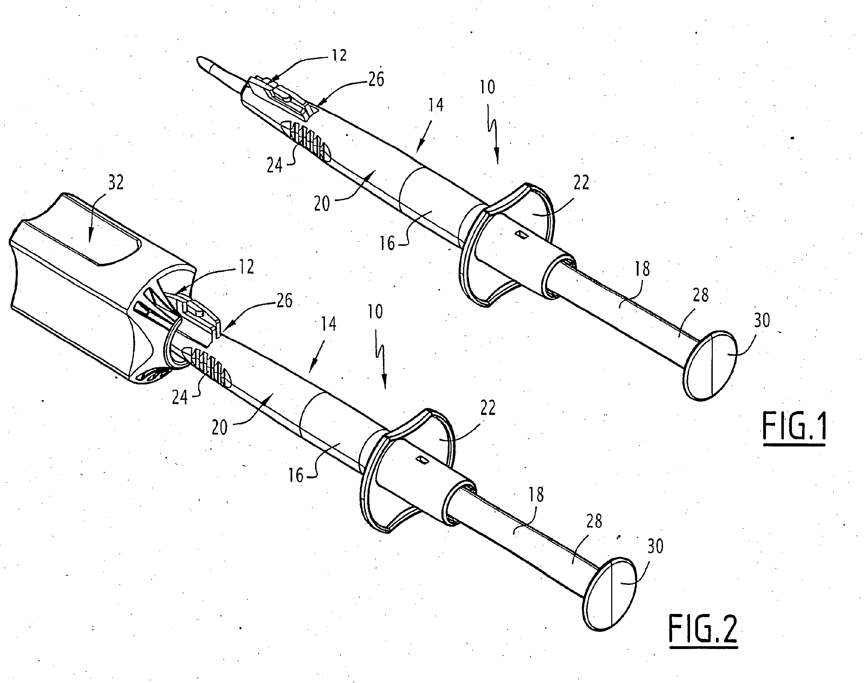

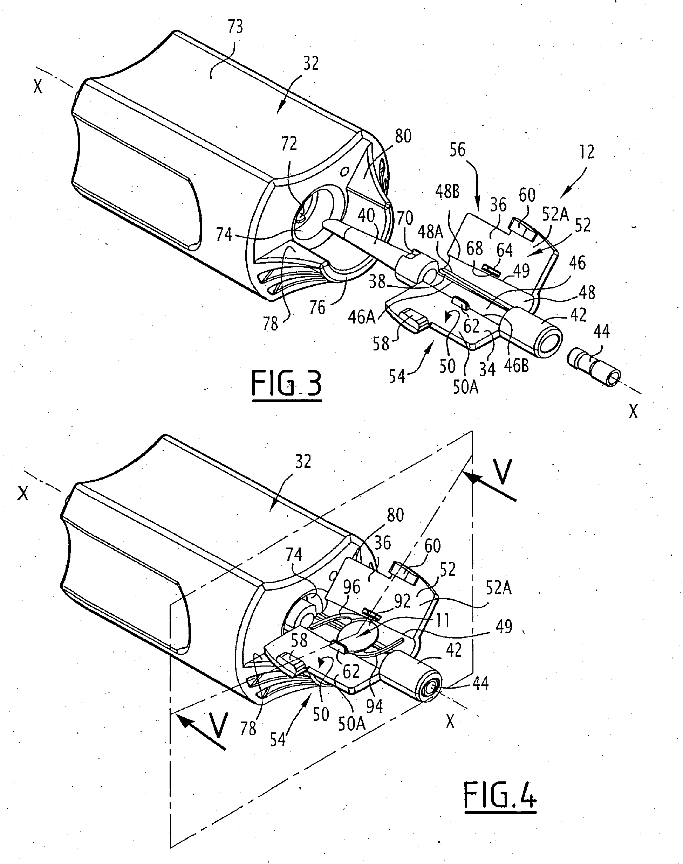

L'injecteur d'implant 10 représenté sur la Figure 1 est destiné à introduire dans l'oeil du patient un implant ophtalmique 11 visible sur la Figure 4 et comprenant notamment une lentille ayant généralement la forme d'un disque.

Comme connu en soi, l'injecteur comporte une cartouche de pliage 12 contenant l'implant à injecter et un mécanisme d'injection 14 formé d'un corps tubulaire 16 présentant à une extrémité des moyens d'accrochage de la cartouche de pliage et un poussoir 18 monté coulissant au travers du corps dans le prolongement de la cartouche de pliage.

Plus précisément, le corps 16 comporte un tube 20 présentant à une extrémité arrière une collerette périphérique 22 formant un appuie-doigt. A son extrémité opposée, le tube 20 présente des empreintes anti-dérapantes 24 facilitant sa préhension. Le corps tubulaire 16 est fendu à son extrémité opposée à celle par laquelle pénètre le poussoir 18 d'une encoche 26 propre à recevoir une excroissance radiale de la cartouche de pliage 12 et permettant une liaison axiale de la cartouche 12 et du corps 16. Ainsi, l'encoche 26 présente à son extrémité débouchant à l'extrémité du tube 20 un passage étroit prolongé par une lumière évasée permettant une liaison par baïonnette de la cartouche 12 et du corps 16.

Le poussoir 18 comporte une tige d'actionnement 28 propre à traverser de part en part le tube 20 et à l'extrémité arrière de la tige 28 une pastille 30 formant appuie-doigt.

Comme illustré sur la Figure 2, l'injecteur comporte en outre un étui de protection 32 de l'extrémité d'injection de la cartouche de pliage. Cet étui est visible avec la cartouche ouverte sur la Figure 3.

La cartouche 12 comporte deux branches 34, 36 articulées l'une par rapport à l'autre autour d'une charnière 38 dont l'axe s'étend parallèlement à l'axe X-X d'injection de l'implant défini par la cartouche. Les deux branches articulées sont prolongées suivant l'axe d'injection X-X d'un côté par une canule 40 et de l'autre côté par un manchon 42 de réception d'un piston d'injection 44 propre à être déplacé suivant l'axe X-X sous l'action du poussoir 18.

Plus précisément, les deux branches 34, 36 présentent chacune au voisinage de l'articulation 38 un canal 46, 48. Ces deux canaux s'étendent parallèlement l'un à l'autre et s'ouvrent l'un en regard de l'autre lorsque les deux branches sont accolées. Ces canaux s'étendent parallèlement à la direction d'articulation de la charnière. Ils ont chacun un section semi-circulaire ou plus précisément semi-elliptique.

Les deux branches sont articulées de sorte que lorsque les branches sont écartées, les deux canaux 46, 48 s'étendent sensiblement parallèlement l'un à l'autre et délimitent transversalement deux cavités successives. En revanche, lorsque les deux branches 34, 36 sont accolées, les deux canaux 46, 48 se superposent pour former un conduit délimitant un contour fermé.

Les canaux 46, 48 sont délimités longitudinalement par des rives sensiblement parallèles. Ainsi, chaque canal est délimité par une rive intérieure 46A, 48A ménagée le long de la charnière 38. Ces rives s'étendent parallèlement l'une à l'autre et sont telles que lorsque les deux branches 34, 36 sont accolées, les deux rives intérieures 46A, 48A sont rigoureusement alignées et confondues, les deux canaux se prolongeant continûment et tangentiellement au niveau des rives intérieures accolées 46A, 48A.

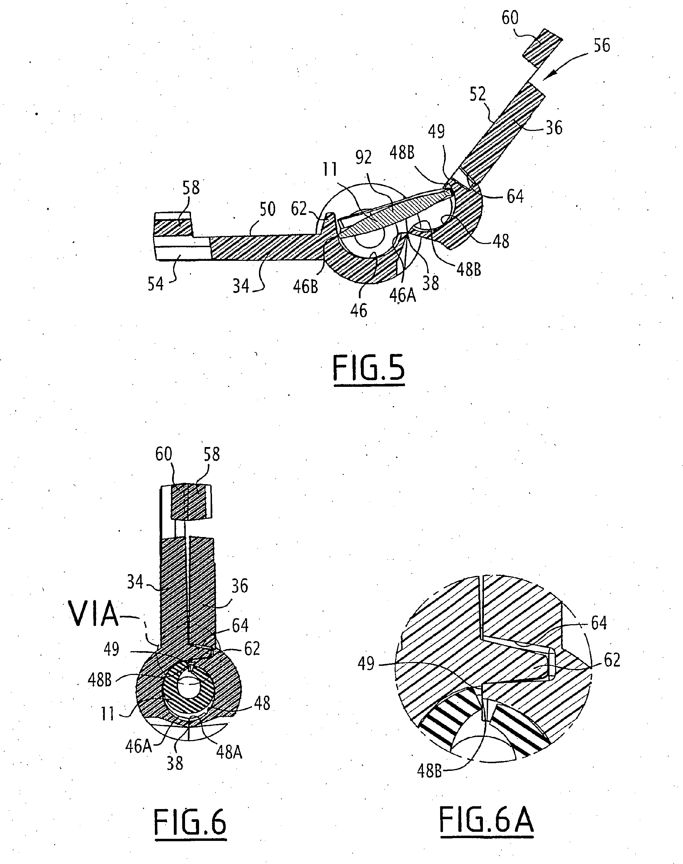

Les deux canaux 46, 48 sont de l'autre côté délimités par deux rives extérieures 46B, 48B ménagées à distance de l'articulation 38. Les rives extérieures 46B, 48B sont décalées transversalement par rapport à l'axe du conduit comme cela sera décrit en détail dans la suite de sorte que lorsque les deux branches sont accolées, les deux rives extérieures 46B, 48B sont décalées l'une à l'autre sans être confondues en formant entre elles un épaulement 49 visible sur la Figure 6A.

En particulier, les deux rives 46B, 48B sont concourantes lorsque les deux branches sont accolées en un point d'extrémité situé immédiatement en sortie du manchon 42. La rive 48B diverge de la rive 46B en direction de la canule 40 en s'écartant de la rive 48B vers l'intérieur du conduit défini par les deux canaux.

Ainsi, l'épaulement 49 est généralement triangulaire ou tropézoïdale, sa largeur augmentant en direction de la canule 40.

Avantageusement, l'épaulement 49 a une largeur moyenne mesurée perpendiculairement à la direction des canaux 46, 48 comprise entre 0,1 mm et 0,8 mm. Plus précisément, et compte tenu de sa forme triangulaire, sa largeur varie de 0 mm au voisinage du manchon 42 à une valeur comprise entre 0,2 et 1 mm et notamment égale à 0,4 mm au niveau de la canule 40.

L'épaulement 49 est plat. Pour ménager l'épaulement 49, les deux canaux ont une largeur différente, les rives 46A, 46B du canal 46 ayant un écartement supérieur aux rives 48A, 48B du canal 48 à l'écart du manchon 42.

Chaque branche 34, 36 présente au-delà du canal 46, 48 associé du côté opposé à la charnière 38 une aile de manoeuvre 50, 52. Ces deux ailes sont de forme générale rectangulaire. Elles présentent l'une en regard de l'autre des surfaces d'appui planes 50A, 52A propres à s'appliquer l'une sur l'autre lorsque les deux branches sont accolées. Ces ailes s'étendent radialement par rapport à la charnière 38. L'épaulement 49 s'étend dans le prolongement de la surface plane 52.

Suivant leur bord libre opposé à la charnière 38, les ailes 50, 52 comportent des encoches respectives 54, 56 disposées tête-bêche. A l'inverse, les ailes 50, 52 comportent sur la surface d'appui 50A, 52A, au droit de l'encoche de l'aile complémentaire des saillies d'encliquetage 58, 60. Ces saillies sont propres à être reçues dans les encoches 54, 56 ménagées sur l'aile en regard. Elles présentent suivant leur extrémité en regard des profils d'enclenchement élastique complémentaires présentant des profils associés en saillie et en creux permettant un maintien des deux branches accolées l'une sur l'autre, les canaux constituant ensemble un conduit à section fermée.

Le long des rives extérieures 46B, 48B des canaux, dans la partie médiane des ailes 50, 52 est prévue une saillie 62 ménagée sur l'aile 50 et une cavité de forme correspondante 64 ménagée dans l'aile 52 est propre à recevoir la saillie 62.

La saillie 62 prolonge la surface du canal 46 à laquelle elle est reliée tangentiellement.

En revanche, la cavité 64 est ménagée à distance de la rive 48B de sorte qu'une partie 66 de l'épaulement 49 s'étend entre la cavité 64 et le canal 48.

Le piston 44 est fixé à l'extrémité du poussoir 18 et est propre à circuler au travers du manchon 42, du conduit délimité par les deux canaux 46, 48 et de la canule 40.

Le manchon 42 et la canule 40 sont ménagés de part et d'autre du canal 42. Elles prolongent celui-ci à chaque extrémité et s'étendent suivant l'axe X-X du canal.

Le conduit intérieur du manchon 42 est de section sensiblement circulaire et constante sur sa longueur.

Le conduit intérieur délimité par la canule 40 est convergeant des canaux 46, 48 vers l'extrémité libre de la canule. Ainsi, intérieurement, la canule présente une surface tronconique de section décroissante des canaux 46, 48 vers l'extrémité d'injection.

Extérieurement, la canule 48 présente deux encoches 70 de positionnement propres à coopérer avec des saillies 72 ménagées dans l'étui de protection.

L'étui de protection 32 est constitué d'un boîtier 73 présentant une ouverture 74 d'introduction de la canule 40 ménagée au centre d'une face d'extrémité. La longueur du boîtier est supérieure à la longueur de la canule. Extérieurement, l'ouverture 74 est bordée sur plus de la moitié de son pourtour par une lèvre 76 présentant à chaque extrémité des surfaces d'appui 78, 80 s'étendant radialement par rapport à l'ouverture 74 et propre à recevoir les ailes 50, 52 de la cartouche lorsque les branches 34, 36 sont écartées comme cela est illustré sur la Figure 4.

La cartouche de pliage 12 est illustrée en position ouverte sur les Figures 4 et 5 alors qu'un implant 11 est disposé entre les canaux 46, 48. Un tel implant présente comme connu en soi une lentille 92 constituée d'un disque transparent et deux bras arqués 94, 96 symétriques l'un de l'autre par rapport à l'axe de la lentille. Les deux bras sont diamétralement opposés par rapport à la lentille.

L'implant est formé dans un matériau polymère déformable élastiquement.

L'implant est mis en forme alors que la canule de la cartouche est maintenue dans l'étui de protection, les deux ailes s'appuyant sur les surfaces 78, 80. La lentille est placée en appui dans les canaux 46, 48 dans leur partie médiane, c'est-à-dire dans la région de la saillie 62 et de l'évidement 64, les deux bras 94, 96 étant disposés de part et d'autre respectivement du côté du manchon 42 et de la canule 40.

L'implant est représenté dans cette position sur la Figure 4.

Dans cette position, l'implant 11 est en contact de part et d'autre avec les surfaces des canaux 46, 48 et prend en appui sur ceux-ci en des points sensiblement diamétralement opposés.

On conçoit que le rapprochement des deux branches 34, 36 par basculement autour de l'articulation 38 provoque le repliement de l'implant 11 autour d'un axe parallèle à l'axe des canaux 46, 48 lorsque ce repliement est initié par le chirurgien appuyant par exemple au moyen d'une pince au centre de l'implant. En particulier, la partie centrale de la lentille se trouve appliquée sur la charnière 38 alors que les bords opposés de la lentille se trouvent rapprochés l'un de l'autre au contact des canaux 46, 48 comme illustré sur la Figure 6 lorsque les deux branches sont accolées.

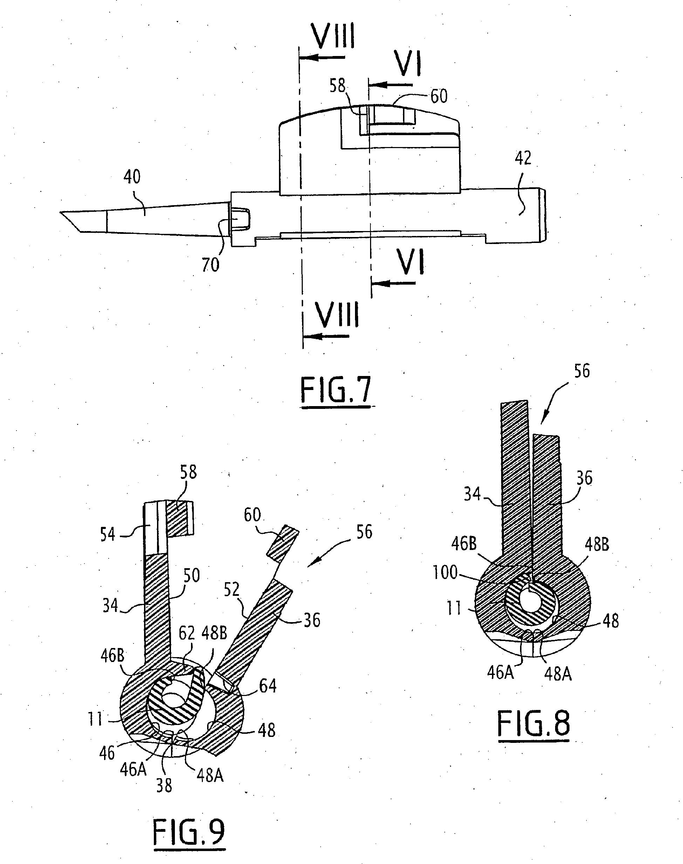

Dans la position et comme illustré sur la Figure 7, les deux branches sont maintenues accolées par coopération des profils d'accrochage 58, 60.

Dans la position de la Figure 6, le bord de la lentille du côté du canal 46 prend appui sur la plage de l'aile 52 formant l'épaulement 49 et faisant saillie à l'intérieur du conduit délimité par les deux canaux 46, 48. En revanche, l'extrémité de la lentille en appui contre le canal 48 se trouve fléchi légèrement à l'intérieur de l'extrémité opposée de la lentille, préfigurant ainsi l'enroulement de la spirale de la lentille.

Comme illustré sur la Figure 8, l'épaulement 49 s'étend sur l'essentiel de la longueur des canaux 46, 48. Ainsi, lorsque la lentille 92 est avancée sous l'action du piston 44 au travers du conduit, les bords opposés de la lentille se chevauchent en garantissant que le bord en appui sur l'épaulement 49 se trouve à l'extérieur du bord recourbé par le canal 48 grâce à l'épaulement 49.

La présence de l'épaulement 49 assure que lors de l'enfoncement de l'implant au travers de la canule, et lors de la poursuite de l'enroulement en spirale de l'implant, les bords opposés soient correctement décalés radialement et superposés, permettant un enroulement satisfaisant de l'implant sur lui-même, sans que les deux bords ne s'appuient l'un sur l'autre. De plus, le sens de roulement de l'implant est garanti puisque imposé par la forme des canaux 46, 48 et la présence de l'épaulement 49.

On conçoit par ailleurs que la présence de la saillie 62 et de l'évidement complémentaire 64 assure qu'en cas de mauvais positionnement initial de l'implant, il soit difficile d'accoler les deux ailes l'une à l'autre comme illustré sur la Figure 9. En effet, si l'un des bords de l'implant s'échappe de l'un des canaux 46, 48, ce bord constitue une barrière interposée entre la saillie 62 et la cavité 64 interdisant que la saillie pénètre dans l'évidement. Ainsi, le chirurgien sent un point dur lors de sa tentative de rapprochement des deux branches et est ainsi informé du mauvais positionnement de l'implant.

En revanche, en cas de positionnement correct de l'implant, comme illustré sur la Figure 7, la saillie 62 est reçue complètement dans la cavité 64.

## Revendications

1. Cartouche (12) de pliage d'implant ophtalmique (11) comportant une pince formée de deux branches articulées (34, 36), les deux branches (34, 36) présentant des canaux (46, 48) sensiblement coaxiaux bordés chacun par deux rives longitudinales (46A, 46B, 48A, 48B), les canaux (46, 48) s'ouvrant l'un en regard de l'autre et délimitant lorsque les deux branches (34, 36) sont accolées un conduit de confinement de l'implant (11) partiellement replié, **caractérisé en ce que** en position accolée des deux branches (34, 36), au moins deux des rives (46B, 48B) en regard des deux canaux (46, 48) sont décalés transversalement au conduit dans le plan de jonction des deux branches (34, 36) de sorte qu'une branche (36) délimite un épaulement (49) d'appui de l'implant (11) faisant saillie en regard du canal (46) ménagé dans l'autre branche (34).

2. Cartouche selon la revendication 1, **caractérisée en ce que** l'épaulement (49) a dans le plan de jonction une largeur moyenne mesurée transversalement à la direction des canaux comprise entre 0,1 et 0,8 mm.

3. Cartouche selon la revendication 1 ou 2, **caractérisée en ce que** les deux rives (46B, 48B) décalées sont plus éloignées de l'axe d'articulation (38) des deux branches (34, 36) que les deux autres rives (46A, 46B), lesquelles autres rives sont confondues lorsque les deux branches (34, 36) sont accolées.

4. Cartouche selon l'une quelconque des revendications précédentes, **caractérisée en ce que** la cartouche comporte une canule d'injection (40) à surface interne convergente dans le prolongement du conduit de confinement de l'implant (11) lorsque les deux branches sont accolées.

5. Cartouche selon l'une quelconque des revendications précédentes, **caractérisée en ce que** les deux canaux (46; 48) sont de section sensiblement semi-circulaire ou semi-elliptique.

6. Cartouche selon l'une quelconque des revendications précédentes, **caractérisée en ce que** l'épaulement (49) est sensiblement plan et l'axe du conduit formé des deux canaux (46, 48) s'étend sensiblement dans le plan défini par l'épaulement (49).

7. Cartouche selon l'une quelconque des revendications précédentes, caractérisée en ce chaque branche (34, 36) présente au-delà du canal (34, 36), du côté opposé à l'axe d'articulation (38), une aile de manoeuvre (50, 52) et en ce que l'épaulement (49) prolonge une aile (52) de manoeuvre de l'une des branches (48).

8. Cartouche selon l'une quelconque des revendications précédentes, **caractérisée en ce que** les deux branches (34, 36) comportent, au voisinage des rives (48/A, 48B) des canaux, en dehors des canaux, des profils d'emboîtement complémentaires (62, 64) en saillie et en creux dimensionnés de sorte que les profils sont emboîtés lorsque les deux branches (34, 36) sont accolées.

9. Injecteur d'implant comportant une cartouche selon l'une quelconque des revendications précédentes et un mécanisme d'injection comprenant un corps de liaison à la cartouche et un poussoir propre à pousser l'implant contenu dans la cartouche.

## Patentansprüche

1. Kassette (12) zum Biegen eines ophthalmischen Implantats (11), aufweisend eine Zange, die aus zwei gelenkig verbundenen Armen (34, 36) gebildet ist, wobei die beiden Arme (34, 36) Kanäle (46, 48) aufweisen, die jeweils im Wesentlichen koaxial begrenzt sind durch zwei Längsbegrenzungen (46A, 467B, 48A, 48B), wobei die Kanäle (46, 48) sich zueinander hin öffnen und, wenn die beiden Arme (34, 36) aneinander angefügt sind, eine Röhre begrenzen zur Umschließung des teilweise gebogenen Implantats, **dadurch gekennzeichnet, dass** in der aneinander angefügten Position der beiden Arme (34, 36) wenigstens zwei der Begrenzungen (46B, 48B) bezüglich der beiden Kanäle (46, 48) quer versetzt sind zur Röhre in der Verbindungsebene der beiden Arme (34, 36), derart, dass ein Arm (36) eine Schulter (49) zur Auflage des Implantats (11) begrenzt, die bezüglich des Kanals (46) vorsteht, der im anderen Arm (34) vorliegt.

2. Kassette gemäß Anspruch 1, **dadurch gekennzeichnet, dass** die Schulter (49) in der Verbindungsebene eine mittlere Breite, die quer zur Richtung der Kanäle gemessen ist, von zwischen 0,1 und 0,8 mm hat.

3. Kassette gemäß Anspruch 1 oder 2, **dadurch gekennzeichnet, dass** die beiden versetzten Begrenzungen (46B, 48B) von der Schwenkachse (38) der beiden Arme (34, 36) weiter entfernt sind als die beiden anderen Begrenzungen (46A, 46B), wobei die anderen Begrenzungen zusammengebracht sind, wenn die beiden Arme (34, 36) aneinander angefügt sind.

4. Kassette gemäß irgendeinem der vorhergehenden Ansprüche, **dadurch gekennzeichnet, dass** die Kassette eine Injektions-Kanüle (40) mit konvergierender Innenfläche in der Verlängerung der Umschließungs-Röhre des Implantats (11) hat, wenn die beiden Arme aneinander angefügt sind.

5. Kassette gemäß irgendeinem der vorhergehenden Ansprüche, **dadurch gekennzeichnet, dass** die beiden Kanäle (46, 48) einen im Wesentlichen halbkreisförmigen oder halbelliptischen Querschnitt haben.

6. Kassette gemäß irgendeinem der vorhergehenden Ansprüche, **dadurch gekennzeichnet, dass** die Schulter (49) im Wesentlichen flach ist und sich die Achse der Röhre, die von den beiden Kanälen (46, 48) gebildet ist, im Wesentlichen in der von der Schulter (49) definierten Ebene erstreckt.

7. Kassette gemäß irgendeinem der vorhergehenden Ansprüche, **dadurch gekennzeichnet, dass** jeder Arm (34, 36) auf der der Schwenkachse (38) entgegengesetzten Seite über den Kanal hinausgehend einen Flügel (52) zum Betätigen des einen der Arme (48) hat.

8. Kassette gemäß irgendeinem der vorhergehenden Ansprüche, **dadurch gekennzeichnet, dass** die beiden Arme (34, 36) benachbart zu den Begrenzungen (48A, 48B) der Kanäle, außerhalb der Kanäle komplementäre Vorsprungs- und Aussparungs-Eingreifprofile (62, 64) haben, die bemessen sind, derart, dass die Profile ineinander eingreifen, wenn die beiden Arme (34, 36) aneinander angefügt sind.

9. Implantat-Injektor mit einer Kassette gemäß irgendeinem der vorhergehenden Ansprüche und einem Injektionsmechanismus mit einem Körper zur Verbindung mit der Kassette und einer Drückvorrichtung, die in der Lage ist, das in der Kassette enthaltene Implantat zu drücken.

## Claims

1. Cartridge (12) for folding an ophthalmic implant (11) comprising a clamp formed by two articulated arms (34, 36), the two arms (34, 36) having substantially coaxial channels (46, 48), each of which is bordered by two longitudinal edges (46A, 46B, 48A, 48B), the channels (46, 48) opening opposite one another and, when the two arms (34, 36) are closed together, defining a conduit for containing the partially folded implant (11), **characterised in that** when two said arms (34, 36) are closed together, at least two of the edges (46B, 48B) facing the two channels (46, 48) are transversely displaced relative to the conduit in the joining plane of said two arms (34, 36) in such a way that one arm (36) defines a shoulder (49) which supports the implant (11) and projects opposite the channel (46) arranged in the other arm (34).

2. Cartridge according to claim 1, **characterised in that** the shoulder (49) is on average between 0.1 and 0.8 mm wide in the joining plane, measured transversely to the direction of the channels.

3. Cartridge according to either claim 1 or claim 2, **characterised in that** the two displaced edges (46B, 48B) are further away from the axis of articulation (38) of the two arms (34, 36) than the two other edges (46A, 36B) which meet when the two arms (34, 36) are closed together.

4. Cartridge according to any one of the preceding claims, **characterised in that** the cartridge comprises an injection cannula (40), of which the internal surface converges inside the extension of the conduit containing the implant (11) when the two arms are closed together.

5. Cartridge according to any one of the preceding claims, **characterised in that** the two channels (46, 48) have a substantially semi-circular or semi-elliptical cross-section.

6. Cartridge according to any one of the preceding claims, **characterised in that** the shoulder (49) is substantially planar and the axis of the conduit formed by the two channels (46, 48) extends substantially into the plane defined by the shoulder (49).

7. Cartridge according to any one of the preceding claims, **characterised in that** each arm (34, 36) has, beyond the channel (34, 36) and on the side opposite the axis of articulation (38), a manoeuvring flange (50, 52), and the shoulder (49) extends a flange (52) for manoeuvring one of the arms (48).

8. Cartridge according to any one of the preceding claims, **characterised in that** the two arms (34, 36) comprise, next to the edges (48A, 48B) of the channels and outside the channels, complementary projecting and hollow connection profiles (62, 64) which are of such a size that the profiles fit together when the two arms (34, 36) are closed together.

9. Implant injector comprising a cartridge according to any one of the preceding claims and an injection mechanism comprising a body for linking to the cartridge and a plunger capable of pushing the implant contained inside the cartridge.
